(19) Europäisches Patentamt
European Patent Office
Office européen des brevets

(11) EP 4 308 066 B1

(12) EUROPEAN PATENT SPECIFICATION

(45) Date of publication and mention of the grant of the patent:
**15.05.2024 Bulletin 2024/20**

(21) Application number: **22723428.3**

(22) Date of filing: **19.04.2022**

(51) International Patent Classification (IPC):
***A61K 8/02*** *(2006.01)* ***A61K 8/19*** *(2006.01)*
***A61K 8/26*** *(2006.01)* ***A61K 8/37*** *(2006.01)*
***A61K 8/41*** *(2006.01)* ***A61K 8/60*** *(2006.01)*
***A61Q 5/04*** *(2006.01)* ***A61Q 5/06*** *(2006.01)*

(52) Cooperative Patent Classification (CPC):
**A61K 8/41; A61K 8/0241; A61K 8/19; A61K 8/26; A61K 8/37; A61K 8/60; A61Q 5/04; A61Q 5/06**

(86) International application number:
**PCT/EP2022/060292**

(87) International publication number:
**WO 2022/228942 (03.11.2022 Gazette 2022/44)**

# (54) RESHAPING COMPOSITION FOR KERATIN FIBERS

UMFORMUNGSZUSAMMENSETZUNG FÜR KERATINFASERN

COMPOSITION DE REMODELAGE POUR FIBRES DE KÉRATINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.04.2021 EP 21170415**

(43) Date of publication of application:
**24.01.2024 Bulletin 2024/04**

(73) Proprietor: **Kao Germany GmbH
64297 Darmstadt (DE)**

(72) Inventors:
- **KAMGUIA, Ruth
64297 Darmstadt (DE)**
- **SCHAEFER, Sabine
64297 Darmstadt (DE)**
- **LIPINSKI, Normen
64297 Darmstadt (DE)**

(74) Representative: **Miller, Tobias
Kao Germany GmbH
Pfungstädter Straße 98-100
64297 Darmstadt (DE)**

(56) References cited:
**WO-A1-2019/074128 JP-A- H1 179 943
JP-A- 2011 148 749**

- **DATABASE GNPD [Online] MINTEL; 30 June 2012 (2012-06-30), Anonymous: "Styling Heat Activated Hair Straightening Lotion", XP002804575,accession no. 1807639**
- **DATABASE GNPD [Online] MINTEL; 30 December 2011 (2011-12-30), Anonymous: "Smoothing Blow Dry Créme", XP002804576, Database accession no. 1682217**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Field of the invention

The present invention is directed to a reshaping composition for keratin fibers, a reshaping process, and kit-of-parts.

### Background of the invention

Current permanent reshaping processes require steps of reducing the hair and later oxidizing the hair. The reducing step employs reducing agents, such as thioglycolic acid, in order to cleave disulphide bonds within the hair and to allow protein chain movement. In a subsequent step the bonds are reformed by addition of an oxidizing composition, typically comprising hydrogen peroxide. In Western countries these well known processes include cold perming and hot perming techniques, whereas, especially in Asian countries, a digital perming as a specialty of the hot perming process is preferred. For a digital perming process the temperature of the perm is controlled by an electronic device, often being equipped with a microprocessor. These multi-step processes are time-consuming (up to 3 hours for a cold perm/5 hours for a digital perm), require a high level of winding skill from the stylist, cause hair damage and, at worst, can lead to hair loss due to over-processing. Over-processing typically stems from either leaving the reducing agent for too long on the hair, or heating the hair to inappropriate temperatures for a too long time. To avoid over-processing, a test curler is typically carried out on a streak of the client's hair prior to the full process being performed, in order to determine the optimum processing time; this adds further to the time needed and requires further skills/education from the stylist. Apart from all the process challenges, the reducing composition has a strong and acrid odour disliked by stylists and consumers alike.

For easing the application of reshaping compositions, WO2016/098870 and WO2019/074128 disclose alkaline non-reducing, non-oxidizing compositions comprising fatty compounds and water-soluble thickening polymers.

Mintel database entries 1807639 and 1682217 disclose hair styling products comprising lipophilic agents and sucrose esters.

However, stability and performance of these compositions still remain challenging.

The first object of the present invention is a cosmetic composition for reshaping keratin fibers with heat, preferably for reshaping human keratin fibers with heat, more preferably for reshaping human hair with heat, having a pH in the range of 7 to 12, and comprising:

a) one or more alkalizing agent(s) selected from

- ammonia and/or ammonium salt(s), and/or
- organic am ine(s) and/or salt(s) of organic am ines according to the following general structure:

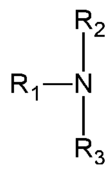

  wherein $R_1$, $R_2$, and $R_3$ are independently selected from H, linear $C_1$-$C_6$ alkyl which may be substituted with one hydroxyl group, or branched $C_3$-$C_{12}$ alkyl or alkanol, wherein at least one of $R_1$, $R_2$, or $R_3$ is different from H, and/or
- mixtures of the alkalizing agents from above,

b) one or more lipophilic com pound(s) at a total concentration in the range of 1% to 80% by weight, calculated to the total weight of the composition,
c) one or more sucrose ester(s).

Preferably, from the viewpoint of cosmetic application the composition is a permanent reshaping composition for reshaping with heat, preferably with heat above 50°C, more preferably with heat at 80°C or above. Most preferably the composition is a permanent waving composition for reshaping with heat, preferably with heat above 50°C, more preferably with heat at 80°C or above.

The second object of the present invention is a process for reshaping keratin fibers, preferably human keratin

fibers, more preferably human hair comprising the steps of:

i) putting keratin fibers under mechanical tension,
ii) applying to keratin fibers the composition as defined above,
iii) optionally covering keratin fibers with a moisture barrier,
iii) optionally covering keratin fibers with a moisture barrier,
iv) heating the keratin fibers to a temperature in the range of 50°C to 230°C,
v) optionally removing the moisture barrier from keratin fibers,
vi) releasing tension from keratin fibers,
vii) optionally rinsing-off the keratin fibers,

wherein process steps i), ii), and vi), vii) can be executed in either order.

The third object of the present invention is a kit-of-parts comprising the composition as defined above and one or more digital perm curler(s), wherein the digital perm curlers are cylindrical hair curlers comprising a heating means.

## Detailed description of the invention

Inventors of the present invention have unexpectedly found out that a composition according to claim 1 has excellent heat stability during perming, high curling efficiency, improved cosmetic safety by preventing dripping at process temperatures above 50°C, and confers cosmetic properties to keratin fibers such as feel, touch, and shine. Moreover, the composition has less to no odor and may be applied without having to cover the keratin fibers with a moisture barrier.

## Reshaping composition

The present invention is directed to a cosmetic composition for reshaping keratin fibers with heat, preferably for reshaping human keratin fibers with heat, more preferably for reshaping human hair with heat, having a pH in the range of 7 to 12, and comprising:

a) one or more alkalizing agent(s) selected from

- ammonia and/or ammonium salt(s), and/or
- organic amine(s) and/or salt(s) of organic amines according to the following general structure:

  wherein $R_1$, $R_2$, and $R_3$ are independently selected from H, linear $C_1$-$C_6$ alkyl which may be substituted with one hydroxyl group, or branched $C_3$-$C_{12}$ alkyl or alkanol,
  wherein at least one of $R_1$, $R_2$, or $R_3$ is different from H, and/or

- mixtures of the alkalizing agents from above,

b) one or more lipophilic com pound(s) at a total concentration in the range of 1% to 80% by weight, calculated to the total weight of the composition,

c) one or more sucrose ester(s).

The composition of present invention may comprise low amounts of reducing agents for stabilizing purposes. Preferably, the composition comprises less than 1% by weight of a reducing agent, preferably it comprises less than 0.1% by weight of a reducing agent, calculated to the total weight of the composition, further more preferably it is free of reducing agents.

The composition of present invention may comprise low amounts of oxidizing agents for stabilizing purposes. Preferably, the composition comprises less than 1% by weight of an oxidizing agent, preferably it comprises less than 0.1% by weight of an oxidizing agent, calculated to the total weight of the composition, further more preferably it is free of oxidizing agents.

It is further preferred from the viewpoint of reshaping durability that the composition from above is a composition for reshaping with heat above 50°C, preferably with heat of 80°C or more.

It is preferred from the viewpoint of keratin fiber reshaping performance that the pH of the composition is 7.5 or more, more preferably 8 or more, further more preferably 8.25 or more.

It is preferred from the viewpoint of keratin fiber reshaping performance, cosmetic safety, and damage to keratin fibers that the pH of the composition is 11 or less, more preferably 10.5 or less, further more preferably 10.0 or less.

For attaining the above-mentioned effects, it is preferred that the pH of the composition is in the range of 7.5 to 11, more preferably 8 to 10.5, further more preferably 8.25 to 10.0.

It is preferred from the viewpoint of rinsability that the composition of the present invention is an aqueous composition. In this respect, it is preferred that the composition of the present invention comprises water at 15% by weight or more, more preferably at 20% by weight or more, further more preferably at 30% by weight or more, still further more preferably at 40% by weight or more, still further more preferably at 50% by weight or more, still further more preferably at 60% by weight or more, still further more preferably at 80% by weight or more, calculated to the total weight of the composition.

It is preferred from the viewpoint of keratin fibers reshaping performance that the composition of the present invention comprises water at 90% by weight or less, more preferably at 85% by weight or less, further more preferably at 75% by weight or less, calculated to the total weight of the composition.

For attaining the above-mentioned effects, it is preferred that the composition of the present invention comprises water in the range of 15% to 90% by weight, more preferably 20% to 85% by weight, further more preferably 30% to 75% by weight, still further more preferably 40% to 75% by weight, still further more preferably 50% to 75% by weight, still further more preferably 60% to 75% by weight, calculated to the total weight of the composition.

The composition of the present invention preferably is an emulsion. Depending on the concentration of compound(s) according to b), it may be presented in the form of an oil-in-water emulsion. However, at higher concentrations of compound(s) according to b), it may also be possible to formulate the composition as an inverse emulsion, i.e. a water-in-oil emulsion.

**Alkalizing agent(s) according to a)**

The composition of the present invention comprises one or more alkalizing agent(s) as compound(s) according to group a) selected from

- ammonia and/or ammonium salt(s), and/or
- organic am ine(s) and/or salt(s) of organic am ines according to the following general structure:

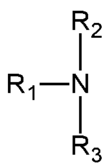

  wherein $R_1$, $R_2$, and $R_3$ are independently selected from H, linear $C_1$-$C_6$ alkyl which may be substituted with one hydroxyl group, or branched $C_3$-$C_{12}$ alkyl or alkanol, wherein at least one of $R_1$, $R_2$, or $R_3$ is different from H, and/or
- mixtures of the alkalizing agents from above.

Suitable ammonium salts are inorganic ammonium and/or organic ammonium salts, and/or their mixtures. Inorganic ammonium salts are preferably selected from ammonium chloride, ammonium sulfate, ammonium carbonate, ammonium hydrogen carbonate, ammonium phosphates, ammonium hydrogen phosphates, ammonium dihydrogen phosphates, ammonium nitrate, ammonium bromide, ammonium iodide, ammonium thiosulfate, ammonium molybdate, ammonium vanadate, and/or their mixtures.

Suitable organic ammonium salts are preferably selected from ammonium carbamate, ammonium sulfamate, ammonium citrate, ammonium salicylate, ammonium valerate, ammonium tartarate, ammonium benzoate, ammonium acetate, ammonium formiate, and ammonium lactate, ammonium salts of polymers, and/or their mixtures.

Preferably, from the viewpoint of buffering, the weight ratio of ammonia to ammonium salt(s) in the composition of the present invention is in the range of 10 to 1.

The organic amine(s) and/or salt(s) of organic amines according to the structure above may be selected from mono- and/or diethanolamine, butyl ethanolamine, butyl diethanolamine, dibutyl ethanolamine, methylethanolamine, triethanolamine, N-lauryl diethanolamine, diisopropanolamine, dimethyl isopropanolamine, isopropanolamine, triisopropanolamine, isobutanolamine, triethylamine, tris-(hydroxymethyl)-aminomethane, and/or aminomethyl propanol. Equally suitable are salts of alkyl and/or alkanol amines with a counterion preferably selected from chloride and/or hydrogen chloride, nitrate, sulphate, phosphate, hydrogenphosphate, dihydrogenphosphate, citrate, acetate, sulphite, benzoate, salicylate.

It is preferred from the viewpoint of keratin fibers reshaping performance and keratin fiber damage that the alkalizing agent according to a) is selected from aminomethyl propanol and/or monoethanolamine, and/or diethanolamine

and/or tris-(hydroxymethyl)-aminomethane, and/or ammonia, and/or their salts, and/or their mixtures.

The most preferred alkalizing agent according to a) of the composition of the present invention is monoethanolamine, ammonia, and tris-(hydroxymethyl)-aminomethane and/or their/its salt(s).

It is preferred from the viewpoint of providing alkalinity and keratin fibers reshaping performance that the total concentration of alkalizing agent(s) in the composition of the present invention is 0.1% by weight or more, preferably 0.5% by weight or more further more preferably 1% by weight or more, calculated to the total weight of the com position.

It is preferred from the viewpoint of keratin fibers reshaping performance and cosmetic safety that the total concentration of alkalizing agent(s) in the composition of the present invention is 25% by weight or less, preferably 20% by weight or less further more preferably 15% by weight or less, calculated to the total weight of the com position.

For attaining the above-mentioned effects, it is preferred that the total concentration of alkalizing agent(s) of the composition of the present invention is in the range of 0.1% to 25% by weight, preferably in the range of 0.5% to 20% by weight, more preferably in the range of 1 %to 15% by weight, calculated to the total weight of the com position.

**Lipophilic compounds according to b)**

The composition of the present invention comprises one or more lipophilic compound(s) at a total concentration in the range of 1% to 80% by weight, calculated to the total weight of the composition, as compound(s) according to b).

It is preferred from the viewpoint of composition stability that the compound(s) according to b) are selected from natural and/or vegetable oils, mineral oil, and fatty acid esters consisting of linear or branched, saturated or unsaturated fatty acids with $C_{12}$ to $C_{22}$ being esterified with linear or branched primary alcohols with $C_3$ to $C_{12}$, silicones, lauryl alcohol, and/or their mixtures.

Suitable natural and/or vegetable oils are, for example, castor oil, avocado oil, olive oil, sunflower oil, rapeseed oil, wheatgerm oil, or almond oil.

Suitable silicones are, for example, linear or cyclic silicones with or without amination, such as dimethicone, trimethicone, and/or amodimethicone.

The preferred compound(s) according to b) are selected from fatty acid esters consisting of linear or branched, saturated or unsaturated fatty acids with $C_{12}$ to $C_{22}$ being esterified with linear or branched primary alcohols with $C_3$ to $C_{12}$, more preferably they are selected from isopropyl palmitate, isopropyl laurate, octyl palmitate, isocetyl palmitate, octyl stearate, oleyl oleate, myristyl myristate, and/or their mixtures, form the viewpoint of curling efficiency and composition stability. The most preferred compound according to b) is isopropyl myristate, isopropyl laurate, cetearyl alcohol, cetyl alcohol, and stearyl alcohol, and/or their mixtures, from the viewpoint of keratin fiber reshaping, removability with washing, and composition stability.

It is preferred from the viewpoint of keratin fibers reshaping performance that the total concentration of compound(s) according to b) is 5% by weight or more, more preferably 20% by weight or more, further more preferably 20% by weight or more, further more preferably 25% by weight or more, calculated to the total weight of the com position.

It is preferred from the viewpoint of removability with washing and composition stability that the total concentration of compound(s) according to b) is 75% by weight or less, more preferably 70% by weight or less, further more preferably 60% by weight or less, still further more preferably 50% by weight or less, still further more preferably 40% by weight or less, calculated to the total weight of the composition.

For attaining the above-mentioned effect, it is preferred that the total concentration of compound(s) according to b) is in the range of 5% to 75% by weight, preferably 10% to 70% by weight, more preferably 15% to 60% by weight, further more preferably 20% to 50% by weight, still further more preferably 25% to 40% by weight, calculated to the total weight of the composition.

**Compounds according to c)**

The composition of the present invention comprises one or more sucrose ester as compound(s) according to group c).

It is preferred from the viewpoint of stabilization performance that compound(s) according to group c) are fatty acid esters of sucrose having one or more $C_{12}$-$C_{22}$ linear or branched, saturated or unsaturated carbon chain(s), which may optionally be substituted with OH.

Suitable compound(s) according to group c) are sucrose stearate, sucrose distearate, sucrose palmitate, sucrose dipalmitate, sucrose laurate, sucrose dilaurate, sucrose myristate, sucrose dimyristate, sucrose behenate, sucrose dibehenate, sucrose oleate, sucrose dioleate, and/or their mixtures, preferably they are sucrose stearate and/or sucrose palmitate, and/or their mixtures, from the viewpoint of heat stability.

It is preferred from the viewpoint of stabilization that the total concentration of compound(s) according to group c) is 0.5% by weight or more, more preferably 1% by weight or more, further more preferably 2% by weight or more, still further more preferably 3% by weight or more, calculated to the total weight of the composition.

It is preferred from the viewpoint of economic reasons that the total concentration of compound(s) according to group c) is 15% by weight or less, more preferably 12% by weight or less, further more preferably 10% by weight or less, still further more preferably 8% by weight or less, calculated to the total weight of the composition.

For attaining the above-mentioned effects, it is preferred that that the total concentration of compound(s) according to group c) is in the range of 0.5% to 15% by weight, preferably 1% to 12% by weight, more preferably in the range of 2% to 10% by weight, more preferably in the range of 3% to 8% by weight, calculated to the total weight of the composition.

**Thickening agent as compound(s) according to group d)**

The composition of the present invention may optionally comprise one or more thickening agent(s) as compound(s) according to group d).

Suitably, one or more compound(s) according to group d) is/are inorganic thickening agent(s), preferably one or more inorganic particulate thickening agent(s), more preferably it is a mineral clay.

Suitable mineral clays are, for example, hectorites and bentonites.

Suitably, one or more compound(s) according to group d) is/are an organic thickening agent(s), preferably one or more thickening polymer(s), more preferably one or more non-ionic, anionic, cationic, or amphoteric/zwitterionic thickening polymer(s).

Suitable anionic thickening polymers are copolymers and/or crosspolymers which comprise an acrylate and/or methacrylate monomer unit and optionally least one more hydrophobic unit such as alkyl chains. Examples are acrylates/c10-30 alkyl acrylate crosspolymer, acrylates/steareth-20 methacrylate copolymer, acrylates/stearyl acrylate/dimethicone methacrylate copolymer, acrylates/beheneth-25 methacrylate copolymer, acrylates/lauryl acrylate/stearyl acrylate/ ethylamine oxide methacrylate copolymer, Hydroxyethyl acrylate / sodium acryloyldimethyl taurate copolymer, carboxymethyl cellulose, alginic acids, sodium alginates, ammonium alginates, calcium alginates, gum arabic, guar gum or xanthan gum, dehydroxanthan gum or acrylic acid polymers known with the CTFA adopted name Carbomer and its derivatives.

Suitable non-ionic thickening polymers are, for example, alkyl modified cellulose derivatives such as ($C_2$-$C_8$)-alkylcellulose, cellulose polymers, preferably methyl cellulose, ethyl cellulose, hydroxyethylcellulose, methylhydroxyethylcellulose, methylhydroxypropylcellulose, or starch based polymers such as vegetable starch and/or their synthetically modified derivatives such as hydroxypropyl starch phosphate.

Suitable cationic non-associative polymers are Polyquaternium 6, Polyquaternium 16, and Polyquaternium 37.

The most preferred thickening polymers of the composition of the present invention are Carbomer and xanthan gum.

In one aspect of the present invention the one or more thickening agent(s) according to group d) is/are thickening agents being soluble in or mixable with the compounds according to b).

The term 'soluble' within the meaning of the present invention denotes the that the compounds according to d) may be solid at 25°C and atmospheric pressure, but are able to completely dissolve in the compounds according to b). Preferably, the complete dissolution appears at 25°C under atmospheric pressure. However, for the purpose of the present invention, it is sufficient that the thickening agent according to d) dissolves in the compounds according to b) at elevated temperatures leading to sufficient thickening of the composition at these temperatures, for example at process temperatures of keratin fiber treatment at 80°C. Hence, it is not required that at 25°C and atmospheric pressure a complete solution of the compounds according to d) is possible to obtain.

The term 'mixable' denotes that the compounds according to d) may be liquid at 25°C and atmospheric pressure. Hence, the compounds according to d) must be at least partially mixable with the compounds according to b) at 25°C under atmospheric pressure. However, complete mixability should be obtained at process temperatures of keratin fiber treatment at 80°C, then delivering the required thickening effect.

In one aspect of the present invention, the oil-soluble thickening agent according to d) is an oil-soluble thickening polymer, preferably a non-ionic, anionic, non-ionic, and/or cationic oil-soluble thickening polymer, more preferably a non-ionic oil-soluble polymer.

Hence, it is preferred for the purpose of the present invention that the compound(s) according to d) are able to thicken the composition of the present invention at a temperature of 80°C or more. Thus, it is preferred from the viewpoint of heat stability of the composition and keratin fiber reshaping performance that the viscosity of the composition at 80°C is 1,000 mPas or more, more preferably 5,000 mPas or more, further more preferably 10,000 mPas or more, measured by plate-cone viscometry, for example with a Brookfield viscometer with appropriate spindle.

It is preferred from the viewpoint of manufacturing that the compounds according to d) are solid or pasty at 25°C under atmospheric conditions.

In one aspect of the present invention, the oil-soluble thickening agent according to d) is an oil-soluble thickening polymer, preferably a non-ionic, anionic, non-ionic, and/or cationic oil-soluble thickening polymer, more preferably a

non-ionic oil-soluble polymer.

It is preferred from the viewpoint of oil solubility of the thickening polymer that the compound(s) according to d) is a homopolymer or copolymer comprising monomer units of ethylene and/or propylene and/or butylene and/or styrene.

Suitable non-ionic polymers satisfying the description of above are, for example, copolymers of ethylene/butylene with styrene monomers are commercially available under the trade name Kraton polymers, for example Kraton G1701E.

In another aspect of the present invention, the compounds according to d) is/are hydrogenated vegetable oil, which is commercially available under the trade name Dermofeel Viscolid from Evonik Ind. AG.

Thus, preferred thickening agents according to d) are homopolymers or copolymers comprising monomer units of ethylene and/or propylene and/or butylene and/or styrene and hydrogenated vegetable oil, and/or their mixtures.

In another aspect of the present invention, the compounds according to d) are lipophilic compounds being solid or pasty at 25°C under atmospheric pressure.

Suitable lipophilic compounds being solid or pasty at 25°C under atmospheric pressure are, for exam ple, fatty alcohols having a linear or branched, saturated or unsaturated $C_{14}$ to $C_{22}$ carbon chain, or hydrogenated oils such as hydrogenated vegetable oils.

Suitably, branched or linear, fatty alcohols having a linear or branched, saturated or unsaturated $C_{14}$ to $C_{22}$ carbon chain are myristyl alcohol, pentadecyl alcohol, cetyl alcohol, palmitoleyl alcohol, heptadecyl alcohol, stearyl alcohol, oleyl alcohol, nonadecyl alcohol, arachidyl alcohol, behenyl alcohol, and/or their mixtures. Such a mixture is cetearyl alcohol comprising stearyl alcohol and cetyl alcohol.

The most preferred thickening agents according to d) are homopolymers or copolymers comprising monomer units of ethylene and/or propylene and/or butylene and/or styrene and hydrogenated vegetable oil, and/or their mixtures.

It is preferred from the viewpoint of thickening effect and reshaping power that the total concentration of compound(s) according to d) is 0.25% by weight or more, more preferably 0.5% by weight or more, further more preferably 0.5% by weight or more, still further more preferably 1% by weight or more, calculated to the total weight of the com position.

It is preferred from the viewpoint of thickening effect, reshaping power, and product safety that the total concentration of compound(s) according to d) is 0.25% by weight or more, more preferably 0.5% by weight or more, further more preferably 1% by weight or more, calculated to the total weight of the composition.

It is preferred from the viewpoint of thickening effect, reshaping power, and rinsability of keratin fibers that the total concentration of compound(s) according to d) is 20% by weight or less, more preferably 15% by weight or less, further more preferably 12% by weight or less, calculated to the total weight of the composition.

For attaining the above-mentioned effects, it is preferred that the total concentration of compound(s) according to d) is in the range of 0.25% to 20% by weight, preferably in the range of 0.5% to 15% by weight, more preferably 1% to 12% by weight, calculated to the total weight of the composition.

**Surfactants other than compound(s) according to group c)**

The composition of the present invention may comprise one or more surfactant(s) other than compound(s) according to group c).

Suitably, such surfactants are anionic, non-ionic, amphoteric and/or zwitterionic, and/or cationic surfactants, and/or their mixtures.

Suitable anionic surfactants are selected from ethoxylated or non-ethoxylated alkyl ether sulfate surfactants, alkyl sulfates, ethoxylated and/or non-ethoxylated alkyl carboxylates, ethoxylated or non-ethoxylated amino acid surfactants, and/or their mixtures.

Suitable alkyl sulfate or preferably ethoxylated alkyl ether sulfate surfactant or mixtures thereof have an alkyl chain length of $C_{10}$ to $C_{22}$.

Suitable example anionic surfactants are laureth sulfates, coceth sulfate, pareth sulfate, capryleth sulphate, myreth sulfate, oleth sulfate, deceth sulfate, trideceth sulfate, coco sulphate, $C_{10}$-$C_{16}$ alkyl sulphate, $C_{11}$-$C_{15}$ alkyl sulphate, $C_{12}$-$C_{18}$ alkyl sulphate, $C_{12}$-$C_{15}$ alkyl sulphate, $C_{12}$-$C_{16}$ alkyl sulphate, $C_{12}$-$C_{13}$ alkyl sulfate, lauryl sulphate, myrystyl sulphate, palm kernel sulphate, cetearyl sulfate, cetyl sulphate, decyl sulphate, oleyl sulphate, behenyl sulphate and/or their salts. All of the aforementioned anionic surfactants may or may not be ethoxylated at various degrees.

Cations for the surfactants may be selected from sodium, potassium, magnesium and/or ammonium.

Suitable non-ionic surfactants are alkyl polyglycosides, ethoxylated triglycerides, ethoxylated fatty alcohols, ethoxylated fatty acid esters, and/or their mixtures.

Preferred non-ionic surfactants are alkyl polyglycosides according to the general structure:

$$R_{23}O(R_{24}O)_tZ_x$$

Wherein Z denotes a carbohydrate with $C_5$ to $C_6$, $R_{23}$ is an alkyl group with $C_8$ to $C_{18}$, $R_{24}$ is methyl, ethyl or

propyl, t ranges from 0 to 10, and x ranges from 1 to 5. Suitable compounds according to this structure are $C_9$-$C_{11}$ alkylpolyglycoside, the structures disclosed in EP-A 70 074, and JP 2015-123019A.

The most preferred compounds according to the structure of above are decyl glucoside, lauryl glucoside, and coco glucoside.

Suitable amphoteric/zwitterionic surfactants are compounds according to the general structure(s)

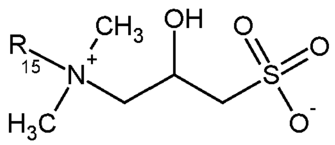

and/or

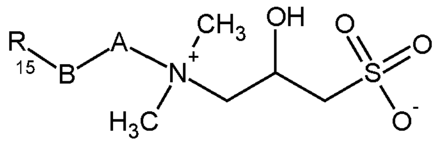

wherein $R_{15}$ is a straight or branched, saturated or unsaturated, substituted or unsubstituted alkyl chain with a carbon number of $C_{10}$ to $C_{22}$, preferably $R_{15}$ is a straight alkyl chain with a carbon number of $C_{10}$ to $C_{16}$, A is a straight alkyl chain with a carbon number of $C_1$ to $C_6$ or a branched alkyl chain with a carbon number of $C_3$ to $C_6$, preferably A is a linear alkyl chain with a carbon number of $C_3$, and B is an amide or an ester group.

Suitable compounds are known as hydroxysultaine surfactants, such as cocoamidopropyl hydroxysultaine, laurylamidopropyl hydroxysultaine, erucamidopropyl hydroxysultaine, lauryl hydroxysultaine, and cocoyl hydroxysultaine, and/or their salt(s).

Further suitable amphoteric/zwitterionic surfactants are of betaine type. Suitable compounds may be selected from alkyl betaines and/or alkylamido betaines. A preferred compound selected from alkyl betaines is lauryl betaine. A preferred compound selected from alkylamido betaines is cocamidopropyl betaine. The disclosure also relates to the salts of the compounds.

The preferred amphoteric/zwitterionic surfactant(s) is/are selected from alkylamido betaines and/or alkylamidoalkyl betaine surfactants.

Suitable cationic surfactants are of quaternary ammonium structure according to the following general structure

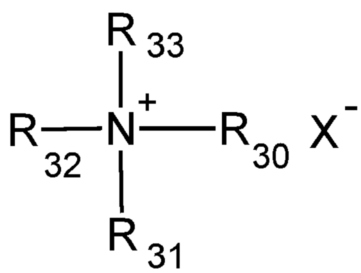

where $R_{30}$ is a saturated or unsaturated, branched or linear alkyl chain with $C_8$-$C_{22}$ or

$R_{34}$ CO NH $(CH_2)_n$

where $R_{34}$ is saturated or unsaturated, branched or linear alkyl chain with $C_7$-$C_{21}$ atoms and n has typical value of 1-4 or

$R_{35}$ CO O $(CH_2)_n$

where $R_{35}$ is saturated or unsaturated, branched or linear alkyl chain with $C_7$-$C_{21}$ atoms and n has typical value of 1-4, and

$R_{31}$ is unsaturated or saturated, branched or linear alkyl chain with $C_1$-$C_{22}$ atoms or

$R_5$ CO NH $(CH_2)_n$

or

$R_6$ CO O $(CH_2)_n$

where $R_{34}$, $R_{35}$ and n are same as above.

$R_{32}$ and $R_{33}$ have an alkyl chain with $C_1$ to $C_4$, and $X^-$ typically is chloride, bromide, or methosulfate.

Typical examples of those ingredients are cetyl trimethyl ammonium chloride, stearyl trimonium chloride, dipalmitoyl dimonium chloride, distearyl dimethyl ammonium chloride, stearamidopropyl trimethyl ammonium chloride, dioleoylethyl dimethyl ammonium methosulfate, dioleoylethyl hydroxyethylmonium methosulfate, behenyl trimethyl ammonium chloride, and/or their mixtures.
It is preferred from the viewpoint of composition stability that the total concentration of surfactants other than compound(s) according to group c) is 0.1% by weight or more, preferably 0.5% by weight or more, further more preferably 0.75% by weight or more, calculated to the total weight of the composition.
It is preferred from the viewpoint of composition stability and cost of goods that the that the total concentration surfactants other than compound(s) according to group c) is 10% by weight or less, further more preferably 7% by weight or less, still further more preferably 5% by weight or less, calculated to the total weight of the com position.
For attaining the above-mentioned effects, it is preferred that the total concentration of surfactants other than compound(s) according to group c) is in the range of 0.1% to 10% by weight, preferably 0.5% to 7% by weight, more preferably in the range of 0.75% to 5% by weight, calculated to the total weight of the composition.

**Process for reshaping keratin fibers**

The present invention is also directed to a process for reshaping keratin fibers, preferably human keratin fibers, more preferably human hair comprising the steps of:

i) putting keratin fibers under mechanical tension,

ii) applying to keratin fibers the composition as defined above,

iii) optionally covering keratin fibers with a moisture barrier,

iv) heating the keratin fibers to a temperature in the range of 50°C to 230°C,

v) optionally removing the moisture barrier from keratin fibers,

vi) releasing tension from keratin fibers,

vii) optionally rinsing-off the keratin fibers,

wherein process steps i), ii), and vi), vii) can be executed in either order.
It is preferred from the viewpoint of reshaping performance that the process is a permanent waving process.
It is preferred form the viewpoint of user convenience that for process step i) the keratin fibers are put under mechanical tension on a curler or roller having heating means. After completion of step ii) or optionally step iii), the curler may then be connected to a digital perm machine for heating the curler and the keratin fibers.
It is preferred from the viewpoint of hair reshaping performance that the keratin fibers are heated in step iv) to a temperature of 80°C or more, more preferably to a temperature of 85°C or more, further more preferably to a temperature of 90°C or more.
It is preferred from the viewpoint of minimizing damage that the keratin fibers are heated in step iv) to a temperature of 180°C or less, more preferably to a temperature of 140°C or less, further more preferably to a temperature of 120°C or less.
For attaining the above mentioned effect, it is preferred that the keratin fibers are heated in step iv) to a temperature in the range of 80°C to 180°C, more preferably 85°C to 140°C, further more preferably 90°C to 120°C.
It is further preferred from the viewpoint of processing time and speedy hair treatment that the heating time of step iv) is 2 min or more, more preferably 5 min or more, further more preferably 10 min or more, still further more preferably 20 min or more.
It is preferred from the viewpoint of keratin fiber reshaping performance and damage reduction that the heating time of step iv) is 60 min or less, more preferably 45 min or less, still further more preferably 40 min or less.
For attaining the above-mentioned effects, it is preferred that the heating time of step iv) is in the range of 2 min to 60 min, more preferably in the range of 5 min to 45 min, further more preferably in the range of 10 min to 40 min, still further more preferably 20 min to 40 min.
It is preferred from the viewpoint of keratin fiber reshaping performance that in step iii) the keratin fibers are covered with a moisture barrier. The moisture barrier of step iii) may be a foil or wrap impermeable for water vapor, or

housing made of a material impermeable for water vapor, with the provision that the selected materials for the moisture barrier are heat resistant up to the selected process temperature.

In principle, many materials are suitable for serving as moisture barrier such as aluminum foil, plastic foil, and/or plastic device, which enclose the curler and/or keratin fiber streak. Alternatively, certain types of anti-flammable fabric is equally suitable. The purpose of the moisture barrier is to keep the keratin fibers moist over the total processing time of heating.

Suitable examples are re-sealable zipper storage bags made of materials such a slow density polyethylene.

For the purpose of the present invention, it is one aspect to cover the keratin fibers with the moisture barrier from the viewpoint of hair reshaping performance. In another aspect from the viewpoint of user convenience, it is preferred that the hair is not covered with a moisture barrier.

**Kit-of-parts**

The present invention is also directed to a kit-of-parts comprising the composition as defined above and one or more digital perm curlers(s), wherein the digital perm curlers are cylindrical hair curlers comprising a heating means.

Suitably, the heating means is an electrical heating means producing heat by its electrical resistance. For connecting such heating means, the curler has electrical connection means at the top and bottom of the cylindrical curler. The curler is thereby means at the top and bottom of the cylindrical curler. The curler is thereby connected to a digital perm machine delivering the electrical power output for the curlers.

Such kit-of-parts may be offered for sale and have the advantage of user convenience, because it includes composition and devices.

It is further preferred that the kit also comprises one or more moisture barrier for covering the curler as defined in optional step ii) above.

The following examples are to illustrate the invention, but not to limit it.

**EXAMPLES**

Compounds according to a) and c) were dissolved in water. The aqueous solution was then heated to 80°C and compound according to b) was added under constant stirring. After cooling the mixture, the pH was adjusted and water balance was added.

| | Ingredients | Comparative examples | | | Working examples | | | | | |
|---|---|---|---|---|---|---|---|---|---|---|
| | | 1 | 2 | 3 | 1 | 2 | 3 | 4 | 5 | 6 |
| | | % by weight [active matter] | | | | | | | | |
| a) | Monoethanolamine | 4.0 | 4.0 | 4.0 | 4.0 | 4.0 | 4.0 | 4.0 | 4.0 | 4.0 |
| b) | Isopropyl myristate | 25.0 | 25.0 | 25.0 | 25.0 | 25.0 | 25.0 | 25.0 | 35.0 | 10.0 |
| - | Coco glucoside* | 5.0 | - | 5.0 | - | - | - | - | - | - |
| - | Ceteareth-20 | - | 5.0 | - | - | - | - | - | - | - |
| c) | Sucrose stearate | - | - | - | 5.0 | - | 3.0 | - | 3.0 | 3.0 |
| c) | Sucrose palmitate | - | - | - | - | 5.0 | - | 3.0 | - | - |
| d) | Carbopol 10** | - | - | 0.5 | - | - | 0.5 | 0.5 | 0.5 | 0.5 |
| - | NaOH/HCl | | | | Ad pH 9.5 | | | | | |
| - | Water | | | | Ad 100.0 | | | | | |
| Evaluati | Storage stability | No | No | No | Yes | Yes | Yes | Yes | Yes | Yes |
| | Dripping at 90°C | Yes | Yes | Yes | No | No | No | No | No | No |
| | Curl ratio L | 9.3 | 8.1 | 8.6 | 13.2 | 12.2 | 14.9 | 11.8 | 12.3 | 10.6 |

* Plantacare 818 sold by BASF Corp.

** Carbopol Ultrez 10 sold by Lubrizol Corp.

**Methods**

Human hair streaks (Caucasian, 21 cm long, 2 g per bundle) were purchased from Fischbach + Miller Haar, Laupheim, Germany. The hair streaks were shampooed with a commercially available shampoo under the brand name

Goldwell Deep Cleansing Shampoo. Then the streaks were towel dried. Then 1 g of the compositions from above was applied to the hair streaks with a brush. The streaks were then wound on perming rods possessing an electrical heating system. The rods were then heated to a temperature in the range of 90°C for 20 min with a digital perming machine. Then the rods were allowed to cool down, and the hair was shampooed with the same shampoo from above. The streaks were then blow-dried.

Assessment of curling efficiency was investigated by measuring and calculating the curl ratio L according to the formula:

$$L = (L_0 - L_t)/L_0$$

wherein $L_0$ is the length of the hair streak prior to curling and Lt is the length of the hair streak after the curling experiment. The number is reported as percentage and a higher percentage corresponds to higher curling degree.

Storage stability test was conducted at 25°C over one week. Judgement of the composition was done by the naked human eye. Instabilities such as phase separations were checked for.

Dripping at 90°C was judges by placing a drop of the composition onto a digital perm curler and heating it to 90°C for 15 min. Judgement was conducted by the naked human eye.

| | **Ingredients** | **Working examples** | | |
|---|---|---|---|---|
| | | **7** | **8** | **9** |
| | | **% by weight [active matter]** | | |
| a) | Monoethanolamine | 0.5 | 4.0 | 4.0 |
| a) | Ammonia (25% v/v) | 0.5 | - | - |
| a) | Tris-(hydroxymethyl)-aminomethane | 5.0 | - | - |
| b) | Isopropyl myristate | 25.0 | 25.0 | 25.0 |
| c) | Sucrose stearate | 3.0 | 3.0 | 3.0 |
| d) | Carbomer | 0.5 | - | - |
| d) | Hectorite | - | 4.0 | - |
| d) | Hydroxyethyl acrylate / sodium acryloyldimethyl taurate copolymer | - | - | 0.5 |
| - | NaOH/HCl | Ad pH 9.5 | | |
| - | Water | Ad 100.0 | | |
| **Evaluation** | Storage stability | Yes | Yes | Yes |
| | Dripping at 90°C | No | No | No |
| | Curl ratio L | 14.9 | 12.3 | 12.1 |

**Claims**

1. A cosmetic composition for reshaping keratin fibers with heat, preferably for reshaping human keratin fibers with heat, more preferably for reshaping human hair with heat, having a pH in the range of 7 to 12, and comprising:

   a) one or more alkalizing agent(s) selected from

- ammonia and/or ammonium salt(s), and/or
- organic amine(s) and/or salt(s) of organic amines according to the following general structure:

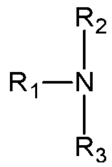

wherein $R_1$, $R_2$, and $R_3$ are independently selected from H, linear $C_1$-$C_6$ alkyl which may be substituted with one hydroxyl group, or branched $C_3$-$C_{12}$ alkyl or alkanol, wherein at least one of $R_1$, $R_2$, or $R_3$ is different from H, and/or
- mixtures of the alkalizing agents from above,

b) one or more lipophilic compound(s) at a total concentration in the range of 1% to 80% by weight, calculated to the total weight of the composition,
c) one or more sucrose ester(s).

2. The composition according to claim 1 **characterized in that** is comprises less than 1% by weight of a reducing agent, preferably it comprises less than 0.1% by weight of a reducing agent, calculated to the total weight of the composition, further more preferably it is free of reducing agents.

3. The composition according to any of the claims 1 and/or 2 **characterized in that** the composition comprises less than 1% by weight of an oxidizing agent, preferably it comprises less than 0.1% by weight of an oxidizing agent, calculated to the total weight of the composition, further more preferably it is free of oxidizing agents.

4. The composition according to any of the preceding claims **characterized in that** the pH is in the range of 7.5 to 11, preferably in the range of 8.0 to 10.5, more preferably in the range of 8.25 to 10.

5. The composition according to any of the preceding claims **characterized in that** the compound(s) according to b) are selected from natural and/or vegetable oils, mineral oil, and fatty acid esters consisting of linear or branched, saturated or unsaturated fatty acids with $C_{12}$ to $C_{22}$ being esterified with linear or branched primary alcohols with $C_3$ to $C_{12}$, and/or silicones, and/or lauryl alcohol, cetearyl alcohol, cetyl alcohol, and stearyl alcohol, and/or their mixtures.

6. The composition according to any of the preceding claims **characterized in that** compound(s) according to b) are isopropyl palmitate, isopropyl laurate, octyl palmitate, isocetyl palmitate, octyl stearate, oleyl oleate, myristyl myristate, and/or their mixtures, preferably it is isopropyl palmitate and/or isopropyl laurate, and/or their mixtures.

7. The composition according to any of the preceding claims **characterized in that** the total concentration of compound(s) according to b) is in the range of 5% to 75% by weight, preferably 10% to 70% by weight, more preferably 15% to 60% by weight, further more preferably 20% to 50% by weight, still further more preferably 25% to 40% by weight, calculated to the total weight of the composition.

8. The composition according to any of the preceding claims **characterized in that** compound(s) according to group c) are fatty acid esters of sucrose having one or more $C_{12}$-$C_{22}$ linear or branched, saturated or unsaturated carbon chain(s), which may optionally be substituted with OH.

9. The composition according to any of the preceding claims **characterized in that** compound(s) according to group c) are sucrose stearate, sucrose distearate, sucrose palmitate, sucrose dipalmitate, sucrose laurate, sucrose dilaurate, sucrose myristate, sucrose dimyristate, sucrose behenate, sucrose dibehenate, sucrose oleate, sucrose dioleate, and/or their mixtures, preferably they are sucrose stearate and/or sucrose palmitate, and/or their mixtures.

10. The composition according to any of the preceding claims **characterized in that** the total concentration of compound(s) according to group c) is in the range of 0.5% to 15% by weight, preferably 1% to 12% by weight, more preferably in the range of 2% to 10% by weight, more preferably in the range of 3% to 8% by weight, calculated to the total weight of the composition.

**11.** The composition according to any of the preceding claims **characterized in that** it comprises one or more thickening agent(s) as compound(s) according to group d).

**12.** The composition according to claim 11 **characterized in that** one or more compound(s) according to group d) is/are inorganic thickening agent(s), preferably one or more inorganic particulate thickening agent(s), more preferably it is a mineral clay.

**13.** The composition according to any of the claims 11 and/or 12 **characterized in that** it one or more compound(s) according to group d) is/are an organic thickening agent(s), preferably one or more thickening polymer(s), more preferably one or more non-ionic, anionic, cationic, or amphoteric/zwitterionic thickening polymer(s).

**14.** A process for reshaping keratin fibers, preferably human keratin fibers, more preferably human hair comprising the steps of:

i) putting keratin fibers under mechanical tension,
ii) applying to keratin fibers the composition as defined in claims 1 to 13,
iii) optionally covering keratin fibers with a moisture barrier,
iv) heating the keratin fibers to a temperature in the range of 50°C to 230°C,
v) optionally removing the moisture barrier from keratin fibers,
vi) releasing tension from keratin fibers,
vii) optionally rinsing-off the keratin fibers,

wherein process steps i), ii), and vi), vii) can be executed in either order.

**15.** A kit-of-parts comprising the composition as defined in any of the claims 1 to 13 and one or more digital perm curler(s), wherein the digital perm curlers are cylindrical hair curlers comprising a heating means.

**Patentansprüche**

**1.** Kosmetische Zusammensetzung zur Umformung von Keratinfasern mit Wärme, vorzugsweise zur Umformung von menschlichen Keratinfasern mit Wärme, noch bevorzugter zur Umformung von menschlichem Haar mit Wärme, mit einem pH-Wert im Bereich von 7 bis 12, und umfassend:

a) ein oder mehrere alkalisierende(s) Mittel ausgewählt aus

- Ammoniak und/oder Ammoniumsalz(e), und/oder
- organische(s) Amin(e) und/oder Salz(e) von organischen Aminen gemäß der folgenden allgemeinen Struktur:

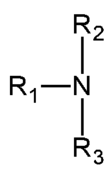

wobei $R_1$, $R_2$ und $R_3$ unabhängig voneinander ausgewählt sind aus H, linearem $C_1$-$C_6$ Alkyl, das mit einer Hydroxylgruppe substituiert sein kann, oder verzweigtem $C_3$-$C_{12}$ Alkyl oder Alkanol, wobei mindestens einer der Reste $R_1$, $R_2$ oder $R_3$ von H verschieden ist, und/oder
- Mischungen der oben genannten Alkalisierungsmittel,

b) eine oder mehrere lipophile Verbindung(en) in einer Gesamtkonzentration im Bereich von 1 bis 80 Gew.-%, bezogen auf das Gesamtgewicht der Zusammensetzung,
c) einem oder mehreren Saccharoseester(n).

**2.** Zusammensetzung nach Anspruch 1, **dadurch gekennzeichnet, dass** sie weniger als 1 Gew.-% eines Reduktionsmittels, vorzugsweise weniger als 0,1 Gew.-% eines Reduktionsmittels, bezogen auf das Gesamtgewicht der

Zusammensetzung, enthält, und weiter bevorzugt frei von Reduktionsmitteln ist.

3. Zusammensetzung nach einem der Ansprüche 1 und/oder 2, **dadurch gekennzeichnet, dass** die Zusammensetzung weniger als 1 Gew.-% eines Oxidationsmittels, vorzugsweise weniger als 0,1 Gew.-% eines Oxidationsmittels, bezogen auf das Gesamtgewicht der Zusammensetzung, enthält und weiter bevorzugt frei von Oxidationsmitteln ist.

4. Zusammensetzung nach einem der vorhergehenden Ansprüche, **dadurch gekennzeichnet, dass** der pH-Wert im Bereich von 7,5 bis 11, vorzugsweise im Bereich von 8,0 bis 10,5, besonders bevorzugt im Bereich von 8,25 bis 10 liegt.

5. Zusammensetzung nach einem der vorhergehenden Ansprüche, **dadurch gekennzeichnet, dass** die Verbindung(en) nach b) ausgewählt sind aus natürlichen und/oder pflanzlichen Ölen, Mineralöl und Fettsäureestern, die aus linearen oder verzweigten, gesättigten oder ungesättigten Fettsäuren mit $C_{12}$ bis $C_{22}$ bestehen, die mit linearen oder verzweigten primären Alkoholen mit $C_3$ bis $C_{12}$ verestert sind, und/oder Silikonen und/oder Laurylalkohol, Cetearylalkohol, Cetylalkohol und Stearylalkohol und/oder deren Mischungen.

6. Zusammensetzung nach einem der vorhergehenden Ansprüche, **dadurch gekennzeichnet, dass** die Verbindung(en) nach b) Isopropylpalmitat, Isopropyllaurat, Octylpalmitat, Isocetylpalmitat, Octylstearat, Oleyloleat, Myristylmyristat und/oder deren Mischungen sind, vorzugsweise ist es Isopropylpalmitat und/oder Isopropyllaurat und/oder deren Mischungen.

7. Zusammensetzung nach einem der vorhergehenden Ansprüche, **dadurch gekennzeichnet, dass** die Gesamtkonzentration der Verbindung(en) nach b) im Bereich von 5 bis 75 Gew.-%, vorzugsweise 10 bis 70 Gew.-%, weiter bevorzugt 15 bis 60 Gew.-%, noch weiter bevorzugt 20 bis 50 Gew.-%, noch weiter bevorzugt 25 bis 40 Gew.-%, bezogen auf das Gesamtgewicht der Zusammensetzung, liegt.

8. Zusammensetzung nach einem der vorhergehenden Ansprüche, **dadurch gekennzeichnet, dass** die Verbindung(en) nach Gruppe c) Fettsäureester von Saccharose mit einer oder mehreren linearen oder verzweigten, gesättigten oder ungesättigten $C_{12}$-$C_{22}$ Kohlenstoffkette(n) sind, die gegebenenfalls mit OH substituiert sein können.

9. Zusammensetzung nach einem der vorhergehenden Ansprüche, **dadurch gekennzeichnet, dass** die Verbindung(en) gemäß Gruppe c) Saccharosestearat, Saccharosedistearat, Saccharosepalmitat, Saccharosedipalmitat, Saccharoselaurat, Saccharosedilaurat, Saccharosemyristat, Saccharosedimyristat, Saccharosebehenat, Saccharosedibehenat, Saccharoseoleat, Saccharosedioleat und/oder deren Mischungen sind, vorzugsweise sind sie Saccharosestearat und/oder Saccharosepalmitat und/oder deren Mischungen.

10. Zusammensetzung nach einem der vorhergehenden Ansprüche, **dadurch gekennzeichnet, daß** die Gesamtkonzentration der Verbindung(en) gemäß Gruppe c) im Bereich von 0,5 bis 15 Gew.-%, vorzugsweise 1 bis 12 Gew.-%, noch bevorzugter im Bereich von 2 bis 10 Gew.-%, noch bevorzugter im Bereich von 3 bis 8 Gew.-%, bezogen auf das Gesamtgewicht der Zusammensetzung, liegt.

11. Zusammensetzung nach einem der vorhergehenden Ansprüche, **dadurch gekennzeichnet, dass** sie ein oder mehrere Verdickungsmittel als Verbindung(en) gemäß Gruppe d) enthält.

12. Zusammensetzung nach Anspruch 11, **dadurch gekennzeichnet, dass** eine oder mehrere Verbindung(en) gemäß Gruppe d) anorganische(s) Verdickungsmittel ist/sind, vorzugsweise ein oder mehrere anorganische(s) teilchenförmige(s) Verdickungsmittel, besonders bevorzugt ein mineralischer Ton.

13. Zusammensetzung nach einem der Ansprüche 11 und/oder 12, **dadurch gekennzeichnet, dass** die eine oder mehrere Verbindung(en) gemäß Gruppe d) ein organisches Verdickungsmittel, vorzugsweise ein oder mehrere verdickende(s) Polymer(e), besonders bevorzugt ein oder mehrere nichtionische(s), anionische(s), kationische(s) oder amphotere/zwitterionische(s) verdickende(s) Polymer(e) ist/sind.

14. Zusammensetzung nach einem der vorhergehenden Ansprüche, **dadurch gekennzeichnet, dass** es sich um eine dauerhafte Umformungszusammensetzung für die Umformung mit Wärme, vorzugsweise mit Wärme über 50°C, noch bevorzugter mit Wärme bei 80°C oder darüber, handelt.

15. Verfahren zur Umformung von Keratinfasern, vorzugsweise von menschlichen Keratinfasern, insbesondere von

menschlichem Haar, umfassend die Schritte:

i) Keratinfasern unter mechanische Spannung setzen,
ii) Auftragen der in den Ansprüchen 1 bis 14 definierten Zusammensetzung auf Keratinfasern,
iii) optionales Abdecken der Keratinfasern mit einer Feuchtigkeitssperre,
iv) Erhitzen der Keratinfasern auf eine Temperatur im Bereich von 50°C bis 230°C,
v) optionales Entfernen der Feuchtigkeitsbarriere von Keratinfasern,
vi) Lösen von Spannungen in den Keratinfasern,
vii) gegebenenfalls Abspülen der Keratinfasern,

wobei die Verfahrensschritte i), ii) und vi), vii) in beliebiger Reihenfolge ausgeführt werden können.

**Revendications**

**1.** Composition cosmétique pour le remodelage à chaud des fibres kératiniques, de préférence pour le remodelage à chaud des fibres kératiniques humaines, plus préférentiellement pour le remodelage à chaud des cheveux humains, ayant un pH compris entre 7 et 12, et comprenant:

a) un ou plusieurs agent(s) alcalinisant(s) choisi(s) parmi

- ammoniaque et/ou sel(s) d'ammonium, et/ou
- amine(s) organique(s) et/ou sel(s) d'amine(s) organique(s) selon la structure générale suivante:

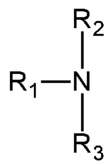

dans lequel $R_1$, $R_2$, et $R_3$ sont indépendamment choisis parmi H, alkyle linéaire $C_1$-$C_6$ qui peut être substitué par un groupe hydroxyle, ou alkyle ramifié $C_3$-$C_{12}$ ou alcool, dans lequel au moins un des $R_1$, $R_2$, ou $R_3$ est différent de H, et/ou
- des mélanges des agents alcalinisants susmentionnés,

b) un ou plusieurs composés lipophiles à une concentration totale comprise entre 1% et 80% en poids, par rapport au poids total de la composition,
c) un ou plusieurs esters de sucrose.

**2.** La composition selon la revendication 1, **caractérisée par le fait qu'**elle comprend moins de 1% en poids d'un agent réducteur, de préférence moins de 0.1% en poids d'un agent réducteur, par rapport au poids total de la composition, de préférence encore elle est exempte d'agents réducteurs.

**3.** La composition selon l'une des revendications 1 et/ou 2, **caractérisée par le fait que** la composition comprend moins de 1% en poids d'un agent oxydant, de préférence moins de 0.1% en poids d'un agent oxydant, par rapport au poids total de la composition, de préférence encore elle est exempte d'agents oxydants.

**4.** La composition selon l'une des revendications précédentes est **caractérisée par le fait que** le pH est compris entre 7.5 et 11, de préférence entre 8.0 et 10.5, plus préférentiellement entre 8.25 et 10.

**5.** La composition selon l'une quelconque des revendications précédentes **caractérisée par le fait que** le(s) composé(s) selon b) sont choisis parmi les huiles naturelles et/ou végétales, les huiles minérales et les esters d'acides gras constitués d'acides gras linéaires ou ramifiés, saturés ou insaturés en $C_{12}$ à $C_{22}$ estérifiés par des alcools primaires linéaires ou ramifiés en $C_3$ à $C_{12}$, et/ou les silicones, et/ou l'alcool laurique, l'alcool cétéarylique, l'alcool cétylique, et l'alcool stéarylique, et/ou leurs mélanges.

**6.** La composition selon l'une des revendications précédentes **caractérisée par le fait que** le(s) composé(s) selon b)

sont le palmitate d’isopropyle, le laurate d’isopropyle, le palmitate d’octyle, le palmitate d’isocétyle, le stéarate d’octyle, l’oléate d’oléyle, le myristate de myristyle, et/ou leurs mélanges, de préférence le palmitate d’isopropyle et/ou le laurate d’isopropyle, et/ou leurs mélanges.

**7.** La composition selon l’une des revendications précédentes est **caractérisée par le fait que** la concentration totale du (des) composé(s) selon b) est comprise entre 5% et 75% en poids, de préférence entre 10% et 70% en poids, plus préférentiellement entre 15% et 60% en poids, encore plus préférentiellement entre 20% et 50% en poids, encore plus préférentiellement entre 25% et 40% en poids, par rapport au poids total de la composition.

**8.** La composition selon l’une quelconque des revendications précédentes, **caractérisée par le fait que** le(s) composé(s) selon le groupe c) sont des esters d’acide gras de sucrose ayant une ou plusieurs chaînes de carbone saturées ou insaturées, linéaires ou ramifiées, en $C_{12}$-$C_{22}$, qui peuvent éventuellement être substituées par OH.

**9.** La composition selon l’une quelconque des revendications précédentes **caractérisée par le fait que** le(s) composé(s) selon le groupe c) sont le stéarate de sucrose, le distéarate de sucrose, le palmitate de sucrose, le dipalmitate de sucrose, le laurate de sucrose, le dilaurate de sucrose, le myristate de sucrose, le dimyristate de sucrose, le béhénate de sucrose, le dibéhénate de sucrose, l’oléate de sucrose, le dioléate de sucrose, et/ou leurs mélanges, de préférence il s’agit du stéarate de sucrose et/ou du palmitate de sucrose, et/ou de leurs mélanges.

**10.** La composition selon l’une des revendications précédentes est **caractérisée par le fait que** la concentration totale de composé(s) selon le groupe c) est comprise entre 0.5% et 15% en poids, de préférence entre 1% et 12% en poids, de préférence entre 2% et 10% en poids, de préférence entre 3% et 8% en poids, calculée par rapport au poids total de la composition.

**11.** La composition selon l’une des revendications précédentes **caractérisée par le fait qu’**elle comprend un ou plusieurs agent(s) épaississant(s) en tant que composé(s) selon le groupe d).

**12.** Composition selon la revendication 11, **caractérisée par le fait qu’**un ou plusieurs composés du groupe d) sont des agents épaississants inorganiques, de préférence un ou plusieurs agents épaississants particulaires inorganiques, plus préférentiellement une argile minérale.

**13.** Composition selon l’une des revendications 11 et/ou 12, **caractérisée par le fait qu’**un ou plusieurs composés du groupe d) sont des agents épaississants organiques, de préférence un ou plusieurs polymères épaississants, plus préférentiellement un ou plusieurs polymères épaississants non ioniques, anioniques, cationiques ou amphotères/zwitterioniques.

**14.** La composition selon l’une quelconque des revendications précédentes est **caractérisée par le fait qu’**il s’agit d’une composition de remodelage permanent pour le remodelage à la chaleur, de préférence à une température supérieure à 50°C, plus préférentiellement à une température de 80°C ou plus.

**15.** Procédé de remodelage des fibres kératiniques, de préférence des fibres kératiniques humaines, et plus particulièrement des cheveux humains, comprenant les étapes suivantes:

i) la mise sous tension mécanique des fibres de kératine,
ii) appliquer sur les fibres kératiniques la composition telle que définie dans les revendications 1 à 14,
iii) recouvrir éventuellement les fibres kératiniques d’une barrière contre l’humidité,
iv) chauffer les fibres de kératine à une température comprise entre 50°C et 230°C,
v) éventuellement, enlever la barrière d’humidité des fibres kératiniques,
vi) relâcher la tension des fibres kératiniques,
vii) rincer éventuellement les fibres kératiniques,

dans lequel les étapes i), ii) et vi), vii) peuvent être exécutées dans n’importe quel ordre.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016098870 A
- WO 2019074128 A
- EP 70074 A
- JP 2015123019 A